# EUROPEAN PATENT APPLICATION

(11) **EP 4 450 008 A1**
(43) Date of publication of application: **23.10.2024**
(21) Application number: 21967758.0
(22) Date of filing: 17.12.2021
(51) Int. Cl.: A61B 17/34

(54) **PUNCTURE POSITIONING SYSTEM AND CONTROL METHOD THEREFOR**

(71) Applicant: Shanghai Droidsurg Medical Co., Ltd, Shanghai 201612 (CN)
(72) Inventor: WANG, Shaobai, Shanghai 201612 (CN); LV, Wener, Shanghai 201612 (CN)
(74) Representative: Bayramoglu et al.
(86) International application number: PCT/CN2021/139241
(87) International publication number: WO 2023/108625

(57) **Abstract**

Puncture positioning system includes planning module, ultrasound module, workstation module, display module, adjustment module and execution module. Puncture positioning system determines operation region and non-operation region by means of MRI image, acquires real-time ultrasound image by means of ultrasound module, and displays reconstructed image information and ultrasound image information of object in real time by means of display module, such that image information can be observed clearly and accurately, and position of operation region can be accurately determined, puncture positioning system establishes operation path by means of workstation module, such that operation path can be more accurate. Puncture positioning system then controls, by means of workstation module, adjustment module to start adjusting position of execution module, such that adjustment precision is higher. And finally, puncture positioning system controls, by means of workstation module, execution module to perform corresponding operation, such that travel distance of execution module can be precisely controlled.

## Description

### TECHNICAL FIELD

The present disclosure relates to the technical field of clinical medical technology, in particular to a puncture positioning system and a control method therefor.

### BACKGROUND

During puncture, a puncture needle is used to pierce a body cavity to extract secretions for testing, inject gas or contrast agents into the body cavity for a contrast examination, or inject a drug into the body cavity, so as to facilitate diagnosis and treatment, such as blood tests, blood transfusion, infusion and catheter inserting for angiography in brain or spinal cavity. For diagnosis or treatment, puncture comprises, but is not limited to, subdural puncture, ventricular puncture, cerebrovascular puncture, lumbar puncture and thoracic cavity puncture and more. In addition, puncture can also be used to construct a channel for following entrance of an operative instrument.

In the prior art, during puncture, a doctor usually acquires image information by means of computed tomography (CT), magnetic resonance imaging (MRI), ultrasound, X-ray, etc., determines a puncture location and a puncture angle with an aid of computer-aided settings, formulates a corresponding surgical plan, before performing the puncture according to a formulated plan. Puncture described above is mainly performed manually relying on a preoperative plan, experience of the doctor and an observed situation, which makes an accuracy of the puncture susceptible to a plurality of factors, makes an accurate location impossible, while increasing learning curves based on clinical imaging for an operator, and enhances risk of an operation.

In addition, on one hand, compared with an ultrasonic guidance, CT has a limited resolution for soft tissue organs, which leads to a blurred display of a boundary between a target anatomical tissue and a surrounding tissue, causes lesions to appear rarely in CT, and results in difficulty in an accurate puncture and intervention operation; while on another hand, MRI has a high resolution for a soft tissue, and therefore can be configured to identify and locate the lesions, and ultrasound can detect an image in a real time. Thus a combination of the ultrasound and the MRI is conducive to achieve an accurate puncture and interventional operation.

### SUMMARY

An objective of the present disclosure is to provide a puncture positioning system and a control method therefor. A clear and accurate image is acquired by a planning module and an ultrasonic module, and displayed in a real time by a display module, while precise control over an adjustment module and an execution module is achieved by a workstation module, thereby accuracy of an operation is improved and harm of an operation to a patient is reduced.

In order to achieve the above objective, the present disclosure provides a puncture positioning system. The puncture positioning system comprises the planning module, the ultrasonic module, the workstation module, the display module, the adjustment module and the execution module, where the planning module is electrically connected to the workstation module, the planning module is applied to acquiring the MRI image of an object and determining an operation area and a non-operation area according to the MRI image, the planning module reconstructs an image according to the operation area, the non-operation area and the MRI image, and the planning module transmits the reconstructed image to the workstation module; the ultrasonic module is electrically connected to the workstation module, and the ultrasonic module is applied to acquiring ultrasonic image of the object and transmitting the ultrasonic image to the workstation module; the workstation module is electrically connected to the display module, the workstation module receives the reconstructed image and the ultrasonic image and constructs an operation path, the workstation module transmits the reconstructed image and the ultrasonic image to the display module, and the display module receives and displays the reconstructed image and the ultrasonic image; and both the adjustment module and the execution module are electrically connected to the workstation module, the workstation module is applied to controlling the adjustment module and the execution module to be switched on and switched off, the execution module is arranged on the adjustment module, the adjustment module is applied to adjusting a location and an angle of the execution module, and the execution module is applied to performing a corresponding operation.

The puncture positioning system of the present disclosure has the beneficial effects: the MRI image of the object is acquired by the planning module, the operation area and the non-operation area of the object are determined according to the MRI image, then the operation area and the non-operation area are divided or outlined, the reconstructed image is acquired to make the operation area and the non-operation area to be displayed more clearly, the ultrasonic image of the object is acquired by the ultrasonic module, the operation path is constructed by the workstation module according to the ultrasonic image and the reconstructed image, the ultrasonic image and the reconstructed image are fused and then transmitted to the display module and displayed by the disply module, then the adjustment module is controlled, by the workstation module, to be switched on and switched off, so as to adjust the location and the angle of the execution module, and finally the corresponding operation is performed. Therefore, according to the puncture positioning system of the present disclosure, the operation area and the non-operation area are determined by means of the magnetic resonance imaging (MRI) image, a real-time ultrasonic image is acquired by the ultrasonic module, and the reconstructed image and the ultrasonic image of the object are fused and displayed in real time by the display module, such that the image can be clearly and accurately observed, and a location of the operation area and a location of the non-operation area can be accurately determined; the operation path can be constructed by the workstation module according to the reconstructed image and the ultrasonic image, such that the operation path is more accurate; then the workstation module controls the adjustment module to be switched on to adjust the location of the execution module, such that higher adjustment accuracy is achieved; and finally, the workstation module controls the execution module to perform the corresponding operation, such that a traveling distance of the execution module can be accurately controlled.

In a feasible solution, the puncture positioning system further comprises a location module, the location module is arranged on the execution module, the location module is electrically connected to the workstation module, and the location module is applied to acquiring the location of the execution module and transmitting the location to the workstation module. The above puncture positioning system has the beneficial effects: by arranging the location module on the execution module, the real-time location of the execution module is acquired and transmitted to the workstation module, such that it is convenient to know the spatial location of the execution module, to accurately move the execution module and to accurately control a relative location of the execution module and the object.

In a feasible solution, the puncture positioning system further comprises a fusion module and a computation module, the fusion module is electrically connected to the workstation module, the display module and the computation module separately, the fusion module acquires the location of the execution module and the operation path and fuses the location and the operation path into the real-time location, and the fusion module transmits the real-time location to the computation module and the display module; and the computation module is electrically connected to the workstation module, and the computation module receives the real-time location, computes a difference between the location of the execution module and the operation path according to the real-time location, and transmits the difference to the workstation module. The above puncture positioning system has the beneficial effects: the location of the execution module and the operation path are fused into the real-time location by the fusion module, the difference between the location of the execution module and the operation path is computed by the computation module and transmitted to the workstation module, such that the workstation module may conveniently determine whether the execution module is adjusted to a location of the operation path according to the difference, and the workstation module can conveniently continue controlling the adjustment module to adjust the location of the execution module according to the difference when the execution module is not adjusted in place.

In a feasible solution, the puncture positioning system further comprises a moving module and a connection module, the workstation module and the display module are arranged on the moving module, the moving module is applied to adjusting a location, the connection module is arranged on the moving module, the ultrasonic module and the adjustment module are arranged on the connection module, and the connection module is applied to adjusting a location of the ultrasonic module and a location of the adjustment module. The above puncture positioning system has the beneficial effects: the location of the puncture positioning system may be conveniently adjusted by the moving module, and fixation and adjustment of spatial location of the execution module and the ultrasonic module may be facilitated by arranging the connection module.

In a feasible solution, the moving module is provided with a hollowed inner cavity, the hollowed inner cavity is applied to storing the connection module, the ultrasonic module, the adjustment module and the execution module. The above puncture positioning system has the beneficial effects: the arrangement facilitates storage of the execution module, the ultrasonic module, the adjustment module and the execution module, on the one hand, occupied space of the puncture positioning system is reduced, and on the other hand, influence of external environment on the above modules may be reduced and aging is slowed down.

In a feasible solution, the planning module comprises an image acquisition unit, an analysis unit, a reconstruction unit and an input/output unit; the image acquisition unit is electrically connected to the analysis unit and the reconstruction unit separately, and the image acquisition unit is applied to acquiring the MRI image and transmitting the MRI image to the analysis unit and the reconstruction unit; the analysis unit is electrically connected to the reconstruction unit, the analysis unit receives the MRI image and determines the operation area and the non-operation area, and the analysis unit transmits information on the operation area and the non-operation area to the reconstruction unit; the reconstruction unit is electrically connected to the input/output unit, and the reconstruction unit receives the MRI image, and the information on the operation area and the non-operation area, reconstructs the image according to the MRI image and the information on the operation area and the non-operation area to acquire the reconstructed image and transmits the reconstructed image to the input/output unit; and the input/output unit is electrically connected to the workstation module, and the input/output unit receives the reconstructed image and transmits the reconstructed image to the workstation module. The above puncture positioning system has the beneficial effects: the MRI image is acquired by the image acquisition unit, the operation area and the non-operation area are determined by the analysis unit, the MRI image, the operation area and the non-operation area are reconstructed by the reconstruction unit, the operation area and the non-operation area are outlined or divided, so as to be displayed more clearly, and the reconstructed image is transmitted to the workstation module by the input/output unit, such that an instruction of the workstation module may be conveniently acquired by the input/output unit.

In a feasible solution, the ultrasonic module comprises an ultrasonic probe, a rotary mechanism, a linear movement mechanism and an ultrasonic sheath; the ultrasonic probe is arranged on the rotary mechanism, the rotary mechanism is applied to driving the ultrasonic probe to rotate, and the ultrasonic probe is applied to acquiring the ultrasonic image; the ultrasonic sheath is arrange on one side of the ultrasonic probe, the rotary mechanism is arranged on the linear movement mechanism, and the linear movement mechanism is applied to driving the ultrasonic probe to reciprocate; and the ultrasonic probe, the rotary mechanism and the linear movement mechanism are electrically connected to the workstation module, the ultrasonic probe transmits the ultrasonic image to the workstation module, and the workstation module controls the ultrasonic probe, the rotary mechanism and the linear movement mechanism to be switched on and switched off separately. The above puncture positioning system has the beneficial effects: friction between the ultrasonic sheath and the object is reduced by arranging the ultrasonic sheath, and the ultrasonic probe may acquire images in different directions or at different portions by arranging the rotary mechanism and the linear movement mechanism.

In a feasible solution, the adjustment module comprises a first moving mechanism, a second moving mechanism, a first fixing clip, a second fixing clip, a third moving mechanism and a first puncture needle guide; the first moving mechanism is arranged on the rotary mechanism, the second moving mechanism is arranged on the first moving mechanism, the first moving mechanism and the second moving mechanism are arranged with an angle defined therebetween, and the first moving mechanism is applied to driving the second moving mechanism to move; the first fixing clip and the third moving mechanism are arranged on the second moving mechanism, and the second moving mechanism is applied to driving the first fixing clip and the third moving mechanism to move; the second fixing clip is arranged on the third moving mechanism, and the third moving mechanism is applied to driving the second fixing clip to rotate around the first fixing clip; the first puncture needle guide is arranged on the first fixing clip and the second fixing clip, and the execution module is arranged on the first puncture needle guide or the third moving mechanism; and the first moving mechanism, the second moving mechanism and the third moving mechanism are all electrically connected to the workstation module, and the workstation module controls the first moving mechanism, the second moving mechanism and the third moving mechanism to be switched on and switched off separately.

The above puncture positioning system has the beneficial effects: the first moving mechanism is fixed by the ultrasonic module, an orientation in space of a plane where the first puncture needle guide is located may be adjusted by the rotary mechanism, a location in space of the plane where the first puncture needle guide is located may be adjusted by the first moving mechanism and the second moving mechanism, the third moving mechanism drives the first puncture needle guide on the second fixing clip to rotate around the first fixing clip, such that the orientation of the first puncture needle guide in the plane may be adjusted, and effects of the rotary mechanism, the first moving mechanism, the second moving mechanism and the third moving mechanism are combined to enable the first puncture needle guide to face any point in the space, and the first puncture needle guide may reach a required path location.

In a feasible solution, the adjustment module comprises a planar moving mechanism, a first angle adjustment mechanism, a second angle adjustment mechanism and a second puncture needle guide, where the first angle adjustment mechanism is arranged on the planar moving mechanism, the second angle adjustment mechanism is arranged on the first angle adjustment mechanism, the first angle adjustment mechanism and the second angle adjustment mechanism are arranged with an angle defined therebetween, the second puncture needle guide is arranged on the second angle adjustment mechanism, the planar moving mechanism is applied to driving the first angle adjustment mechanism to move, the first angle adjustment mechanism is applied to driving the second angle adjustment mechanism to rotate, and the second angle adjustment mechanism is applied to driving the second puncture needle guide to rotate; and the planar moving mechanism, the first angle adjustment mechanism and the second angle adjustment mechanism are each electrically connected to the workstation module, and the workstation module controls the planar moving mechanism, the first angle adjustment mechanism and the second angle adjustment mechanism to be switched on and switched off separately.

The above puncture positioning system has the beneficial effects: a location of the first angle adjustment mechanism is adjusted by arranging the planar moving mechanism, an orientation in space of the second puncture needle guide is adjusted by means of joint effects of the first angle adjustment mechanism and the second angle adjustment mechanism, and the second puncture needle guide may face any point in space by combining the action of the planar moving mechanism.

The present disclosure further provides a control method for the puncture positioning system. The control method comprises: S 1: acquiring a magnetic resonance imaging (MRI) image of an object by a planning module; S2: determining an operation area and a non-operation area of the object on the basis of the MRI image, dividing the operation area and the non-operation area, and reconstructing an image; S3: acquiring an ultrasonic image of the object by an ultrasonic module; S4: fusing the reconstructed image and the ultrasonic image by a workstation module, transmitting a fused image to a display module, and displaying the fused image by the display module; and S5: constructing an operation path by the workstation module, and controlling, by the workstation module, an adjustment module to adjust a location of an execution module to a location of the operation path, and controlling the execution module to perform a corresponding operation.

The control method for the puncture positioning system of the present disclosure has the beneficial effects: the MRI image of the object is acquired by the planning module, the operation area and the non-operation area are determined according to the MRI image, the operation area and the non-operation area are divided, and then the image is reconstructed according to information on the divided operation area, information on the divided non-operation area and the MRI image, such that the operation area and the non-operation area may be displayed more clearly; in addition, the ultrasonic image of the object may be acquired by the ultrasonic module, the reconstructed image and the ultrasonic image may be fused by the workstation module, and are displayed in real time by the display module, facilitating observation by an operator; and then, the operation path may be constructed by the workstation module, and the execution module may be adjusted in location according to the operation path, and is adjusted in place to perform the corresponding operation, such that more accurate location adjustment may be achieved, and operation accuracy may be improved.

In a feasible solution, S5 specifically comprises: S51: constructing several operation paths by the workstation module; S52: selecting one of the operation paths in S51 by the workstation module; S53: controlling the adjustment module to adjust the location of the execution module to a location of the selected operation path; S54: performing the corresponding operation by the execution module; and S55: repeating steps S52-S54 until all operations are completed. The above control method for the puncture positioning system has the beneficial effects: by constructing the several operation paths and selecting a plurality of different operation paths for performing the corresponding operation, pathological samples in the operation area may be acquired at different angles, and more accurate pathological analysis results may be acquired.

In a feasible solution, S53 specifically comprises: S530: providing a location module, where the location module is arranged on the execution module, and the location module acquires a location of the execution module in real time and transmits the location to the workstation module and the display module; and S531: computing, by the workstation module, adjustment and an adjustment distance required by the execution module according to the real-time location and the selected operation path, controlling the adjustment module to be switched on according to the adjustment, controlling the adjustment module to be switched off when the adjustment distance is zero, and receiving and displaying in real time the location by the display module. The above control method for the puncture positioning system has the beneficial effects: the location of the execution module is acquired by the location module, and the adjustment and the adjustment distance required by the execution module are computed by the workstation module, such that the adjustment module may be accurately controlled to be switched on and switched off and the location of the execution module may be accurately adjusted.

In a feasible solution, S54 specifically comprises: S540: with the executing module comprising an execution unit and a drive unit, arranging the execution unit at a movable end of the drive unit, where the execution unit is applied to performing the corresponding operation, the drive unit is applied to driving the execution unit to move, and the workstation module is applied to controlling the drive unit to be switched on and switched off; S541: controlling, by the workstation module, the drive unit to be switched on; S542: acquiring an initial location of the execution unit by the location module, and computing, by the workstation module, a required traveling depth of the execution unit according to the initial location and the operation area; S543: acquiring a real-time traveling distance of the execution unit by the location module, and controlling the drive unit to be switched off when the traveling distance is equal to the depth; S544: performing the corresponding operation by the execution unit; and S545: controlling, by the workstation module, the drive unit to drive the execution unit to exit. The above control method for the puncture positioning system has the beneficial effects: the real-time traveling distance of the execution unit is acquired by the location module, and the drive unit is accurately switched on and switched off according to a traveling depth computed by the workstation module, such that it is guaranteed the execution unit moves to the operation area and acquires the required pathological samples.

In a feasible solution, S3 specifically comprises: S31: switching on the ultrasonic module; S32: moving the ultrasonic module to an area to be operated of the object; and S33: automatically operating the ultrasonic module to acquire the ultrasonic image. The above control method for the puncture positioning system has the beneficial effects: the ultrasonic module is manually moved to the area to be detected, and the ultrasonic module is controlled to rotate or move back and forth by automatic control to acquire the ultrasonic image, such that a use manner is simple, and energy of the operator may be saved conveniently.

In a feasible solution, after S3 and before S4, the control method further comprises: dividing and reconstructing, by the ultrasonic module, the non-operation area according to the ultrasonic image acquired in S3. The above control method for the puncture positioning system has the beneficial effects: through the arrangement, the location of the non-operation area may be re-determined during clinical operation, thereby avoiding damage to the non-operation area.

### BRIEF DESCRIPTION OF DRAWINGS

Fig. 1 is a partial structural schematic diagram of a puncture positioning system in a first embodiment of the present disclosure;
Fig. 2 is a structural block diagram of the puncture positioning system in the first embodiment of the present disclosure;
Fig. 3 is a schematic diagram of an assembling structure of an adjustment module and an ultrasonic module in Fig. 1;
Fig. 4 is a schematic diagram of an assembling structure of an adjustment module and an ultrasonic module in a second embodiment of the present disclosure;
Fig. 5 is a flowchart of a control method for a puncture positioning system in a third embodiment of the present disclosure.

Reference numerals in the figures:
1. planning module;
2. ultrasonic module; 201. ultrasonic probe; 202. rotary mechanism; 203. linear movement mechanism; 204. ultrasonic sheath;
3. workstation module;
4. display module;
5. adjustment module; 501. first moving mechanism; 502. second moving mechanism; 503. first fixing clip; 504. second fixing clip; 505. third moving mechanism; 506. first puncture needle guide; 507. planar moving mechanism; 508. first angle adjustment mechanism; 509. second angle adjustment mechanism; 510. second puncture needle guide;
6. execution module;
7. moving module;
8. connection module.

### DETAILED DESCRIPTION OF THE EMBODIMENTS

To make objectives, technical solutions, and advantages of the present disclosure clearer, the technical solutions in the embodiments of the present disclosure will be clearly and completely described below with reference to the accompanying drawings of the present disclosure. Apparently, the described embodiments are some rather than all of the embodiments of the present disclosure. All other embodiments obtained by a person of ordinary skill in the art based on the embodiments of the present disclosure without creative efforts shall fall within the protection scope of the present disclosure. Unless otherwise defined, technical terms or scientific terms used herein should have ordinary meanings understood by those with ordinary skills in the field to which the present disclosure belongs. As used herein, similar words such as "comprise" mean that elements or objects appearing before the word cover elements or objects listed after the word and their equivalents, but do not exclude other elements or objects.

In order to solve the problems in the prior art, the embodiments of the present disclosure provide a puncture positioning system.

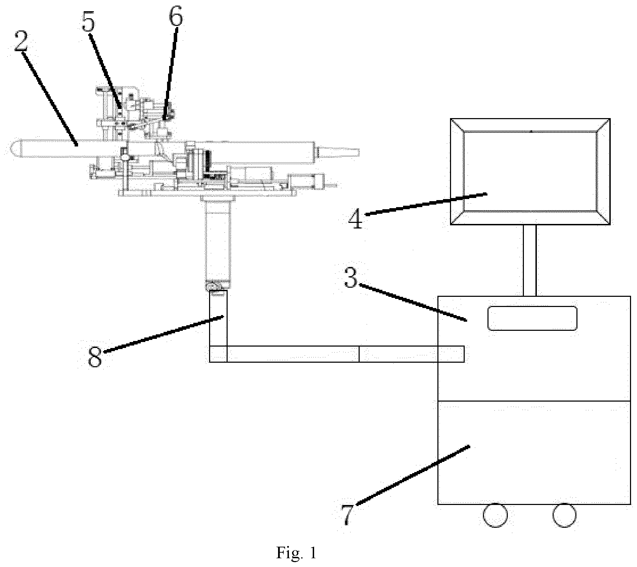

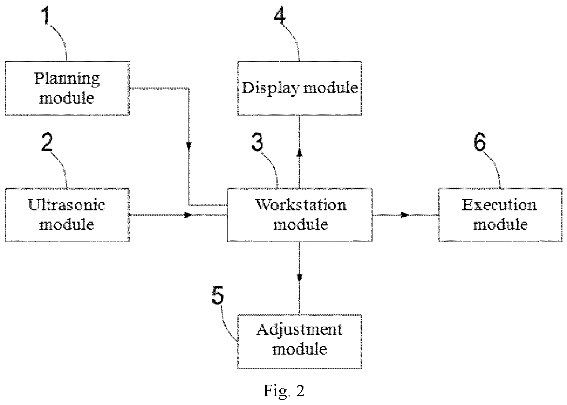

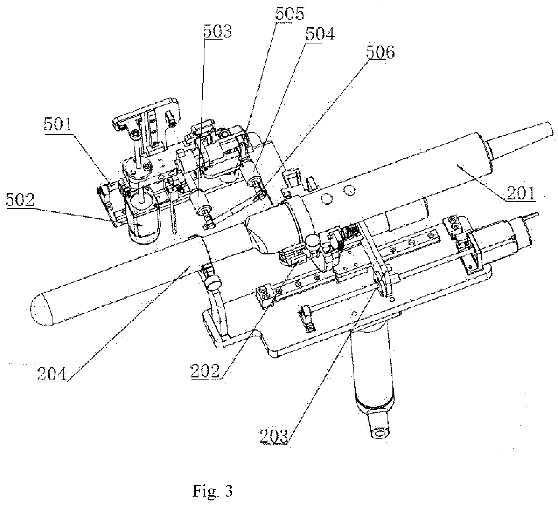

Fig. 1 is a partial structural schematic diagram of a puncture positioning system in a first embodiment of the present disclosure, Fig. 2 is a structural block diagram of the puncture positioning system in the first embodiment of the present disclosure, and Fig. 3 is a schematic diagram of an assembling structure of an adjustment module and an ultrasonic module in Fig. 1.

In some embodiments of the present disclosure, with reference to Figs. 1-3, the puncture positioning system comprises a planning module 1, an ultrasonic module 2, a workstation module 3, a display module 4, an adjustment module 5 and an execution module 6, where the planning module 1 is electrically connected to the workstation module 3, the planning module 1 is applied to acquiring an MRI image of an object and determining an operation area and a non-operation area according to the MRI image, the planning module 1 reconstructs an image according to the operation area, the non-operation area and the MRI image, and the planning module 1 transmits the reconstructed image to the workstation module 3. The ultrasonic module 2 is electrically connected to the workstation module 3, and the ultrasonic module 2 is applied to acquiring an ultrasonic image of the object and transmitting the ultrasonic image to the workstation module 3. The workstation module 3 is electrically connected to the display module 4, the workstation module 3 receives the reconstructed image and the ultrasonic image and constructs an operation path, the workstation module 3 transmits the reconstructed image and the ultrasonic image to the display module 4, and the display module 4 receives and displays the reconstructed image and the ultrasonic image. And both the adjustment module 5 and the execution module 6 are electrically connected to the workstation module 3, the workstation module 3 is applied to controlling the adjustment module 5 and the execution module 6 to be switched on and switched off, the execution module 6 is arranged on the adjustment module 5, the adjustment module 5 is applied to adjusting a location and an angle of the execution module 6, and the execution module 6 is applied to performing a corresponding operation.

In some specific embodiments of the present disclosure, the planning module 1 and the workstation module 3 are connected by means of a cable, the ultrasonic module 2 is arranged in the workstation module 3, and the ultrasonic module 2 comprises an ultrasonic probe 201, a rotary mechanism 202, a linear movement mechanism 203 and an ultrasonic sheath 204, the ultrasonic sheath 204 being fixedly arranged at a front end of the ultrasonic probe 201 and the ultrasonic sheath 204 being hollowed. The front end of the ultrasonic probe 201 extends to a hollowed portion of the ultrasonic sheath 204, the ultrasonic sheath 204 is used to be placed in a body of the object, the ultrasonic probe 201 is arranged in the rotary mechanism 202, the rotary mechanism 202 is arranged on the linear movement mechanism 203, the ultrasonic probe 201 is applied to acquiring the ultrasonic image, the rotary mechanism 202 is applied to driving the ultrasonic probe 201 to rotate, the linear movement mechanism 203 is applied to driving the rotary mechanism 202 to move back and forth, the ultrasonic probe 201, the rotary mechanism 202 and the linear movement mechanism 203 are electrically connected to the workstation module 3, and the workstation module 3 controls the ultrasonic probe 201, the rotary mechanism 202 and the linear movement mechanism 203 to be switched on and switched off separately. The workstation module 3 is a host, and the host is connected to a mouse keyboard for inputting control instructions. The display module 4 is arranged on the workstation module 3, and the display module 4 is electrically connected to the workstation module 3.

The adjustment module 5 comprises a first moving mechanism 501, a second moving mechanism 502, a first fixing clip 503, a second fixing clip 504, a third moving mechanism 505 and a first puncture needle guide 506, where the first moving mechanism 501 is arranged on the rotary mechanism 202, the second moving mechanism 502 is arranged on the first moving mechanism 501, the first moving mechanism 501 is perpendicular to the second moving mechanism 502, the first moving mechanism 501 is applied to driving the second moving mechanism 502 to move, the first fixing clip 503 and the third moving mechanism 505 are arranged on the second moving mechanism 502, the second moving mechanism 502 is applied to driving the first fixing clip 503 and the third moving mechanism 505 to move, and the second fixing clip 504 is arranged on the third moving mechanism 505, the third moving mechanism 505 is applied to driving the second fixing clip 504 to rotate around the first fixing clip 503, the first puncture needle guide 506 is arranged on the first fixing clip 503 and the second fixing clip 504, the execution module 6 is arranged on the first puncture needle guide 506 or the third moving mechanism 505, and the execution module 6 comprises an execution unit (not shown in the figure) and a drive unit (not shown in the figure), the execution unit being a puncture needle, an ablation needle, an intervertebral foramen endoscope, etc. With the puncture needle as an example in the present disclosure, the execution unit is arranged in the first puncture needle guide 506, the drive unit is a drive motor, and the drive motor may drive the execution unit to move in a direction of the first puncture needle guide 506. The first moving mechanism 501, the second moving mechanism 502 and the third moving mechanism 505 are all electrically connected to the workstation module 3, and the workstation module 3 controls the first moving mechanism 501, the second moving mechanism 502 and the third moving mechanism 505 to be switched on and switched off separately. A location of the first moving mechanism 501 and a location of the second moving mechanism 502 are adjusted by the rotary mechanism 202, a location of a plane where the first moving mechanism 501 or the second moving mechanism 502 is located may be adjusted, a location of the first puncture needle guide 506 mapped on the plane may be adjusted by the first moving mechanism 501 and the second moving mechanism 502, an angle of the first puncture needle guide 506 mapped on the plane may be adjusted by the third moving mechanism 505, such that the first puncture needle guide 506 may face any point in the space, and an angle of the first puncture needle guide 506 may be adjusted.

When in use, the MRI image of the object is acquired by the planning module 1. Since generation of the MRI image is slow, the MRI image may be acquired and stored in advance, and then called out for use when necessary. Further, the operation area and non-operation area of the object are determined by means of the MRI image, and the operation area and non-operation area are divided or outlined to make the operation area and non-operation area to be displayed more obviously. Then, the image is reconstructed according to the operation area, the non-operation area and the MRI image, and the reconstructed image is transmitted to the workstation module 3. In this case, the workstation module 3 controls the ultrasonic probe 201 to be switched on, and then controls the rotary mechanism 202 and/or the linear movement mechanism 203 to be switched on, and acquires the ultrasonic image and transmits the ultrasonic image to the workstation module 3. By means of the workstation module 3, the ultrasonic image and the reconstructed image are fused, the non-operation area and operation area of the object are determined, the operation path is constructed, and in this case, the ultrasonic image and the reconstructed image are transmitted to the display module 4 and are displayed by the display module, to be observed by an operator. Then, the location of the execution module 6 is adjusted according to the operation path, so as to make the first puncture needle guide 506 and the operation path collinear. Finally, the workstation module 3 controls the execution module 6 to be switched on to perform the corresponding operation. Therefore, the puncture positioning system in this embodiment may clearly determine the operation area and non-operation area of the object by combining the MRI image and the ultrasonic image, the display module 4 is applied to displaying, facilitating clear observation by the operator, and the feasible operation paths may be constructed according to the operation area and the non-operation area, thereby improving a safety degree of operation. Then, the workstation module 3 controls the adjustment module 5 and the execution module 6 to be switched on and switched off, the execution module is adjusted in location to perform the corresponding operation, and the workstation controls adjustment and operation, thereby reducing errors caused by personnel and making operation accuracy higher.

In some embodiments of the present disclosure, with reference to Figs. 1 and 2, the puncture positioning system further comprises a location module (not shown in the figure), the location module is arranged on the execution module 6, the location module is electrically connected to the workstation module 3, and the location module is applied to acquiring the location of the execution module 6 and transmitting the location to the workstation module 3.

In some specific embodiments of the present disclosure, the location module (not shown in the figure) is arranged on the execution module 6, and the location module is a location device. The location module acquires locations of at least two points on the execution module 6, determines the location and a direction in space of the execution module 6 according to the locations, and may determine a relative location of the execution module 6 and the object, thereby conveniently adjusting the location of the execution module 6 and improving accuracy of location adjustment.

In some embodiments of the present disclosure, with reference to Figs. 1 and 2, the puncture positioning system further comprises a fusion module (not shown in the figure) and a computation module (not shown in the figure), the fusion module is electrically connected to the workstation module 3, the display module 4 and the computation module separately, the fusion module acquires the location of the execution module 6 and the operation path and fuses the location and the operation path into the real-time location, and the fusion module transmits the real-time location to the computation module and the display module 4; and the computation module is electrically connected to the workstation module 3, and the computation module receives the real-time location, computes a difference between the location of the execution module 6 and the operation path according to the real-time location, and transmits the difference to the workstation module 3.

In some specific embodiments of the present disclosure, the fusion module is electrically connected to the display module 4 and the workstation module 3 separately, the fusion module acquires the location of the execution module 6 and the operation path and fuses the location and the operation path into the real-time location, and the display module generates a real-time virtual image according to the real-time location, the MRI image and the ultrasonic image, facilitating observation. The computation module is electrically connected to the fusion module, and the computation module acquires the real-time location and computes the current distance difference between the execution module 6 and the destination operation path. When the difference is equal to zero, it indicates that the execution module 6 is adjusted in place. When the difference is not equal to zero, it indicates that the execution module 6 is not adjusted in place. In this case, a direction difference between the execution module 6 and the operation path may be computed according to the real-time location, facilitating further adjustment.

In addition, the workstation module 3 may reconstruct an image of the location module according to the location, fuse the image into the reconstructed image and/or the ultrasonic image, and transmit the fused image to the display module 4 for real-time display, such that the operator may observe the location of the execution module 6 in real time by the display module 4.

In some embodiments of the present disclosure, with reference to Figs. 1 and 2, the puncture positioning system further comprises a moving module 7 and a connection module 8, the workstation module 3 and the display module 4 are arranged on the moving module 7, the moving module 7 is applied to adjusting a location, the connection module 8 is arranged on the moving module 7, the ultrasonic module 2 and the adjustment module 5 are arranged on the connection module 8, and the connection module 8 is applied to adjusting a location of the ultrasonic module 2 and a location of the adjustment module 5.

In some specific embodiments of the present disclosure, the moving module 7 comprises a rack, a bottom side surface of the rack is provided with several rolling wheels, the display module 4 is arranged on an upper side of the moving module 7, the host is arranged in the moving module 7, the keyboard is fixedly arranged on a shell of the moving module 7, the connection module 8 is a mechanical arm or a universal arm, the connection module 8 is manually adjusted, one end of the connection module 8 is fixedly or rotatably arranged on the moving module 7, and the rotary mechanism 202 is arranged on the other end of the connection module 8. When in use, the puncture positioning system is moved to a required location by the moving module 7, and the location of the ultrasonic module 2 and the location of the adjustment module 5 are adjusted to the area to be operated by the connection module 8, facilitating operation preparation.

In some embodiments of the present disclosure, with reference to Figs. 1 and 2, the moving module 7 is provided with a hollowed inner cavity, the hollowed inner cavity is applied to storing the connection module 8, the ultrasonic module 2, the adjustment module 5 and the execution module 6.

In some specific embodiments of the present disclosure, the hollowed inner cavity is provided at a lower side of the display module 4, one end, close to the workstation module 3, of the connection module 8 is fixedly or movably arranged in the hollowed inner cavity, and the hollowed inner cavity is provided to facilitate storage of the connection module 8, the ultrasonic module 2, the adjustment module 5 and the execution module 6 and adjustment of the location of the puncture positioning system.

In some embodiments of the present disclosure, with reference to Fig. 2, the planning module 1 comprises an image acquisition unit (not shown in the figure), an analysis unit (not shown in the figure), a reconstruction unit (not shown in the figure) and an input/output unit (not shown in the figure). The image acquisition unit is electrically connected to the analysis unit and the reconstruction unit separately, and the image acquisition unit is applied to acquiring the MRI image and transmitting the MRI image to the analysis unit and the reconstruction unit. The analysis unit is electrically connected to the reconstruction unit, the analysis unit receives the MRI image and determines the operation area and the non-operation area, and the analysis unit transmits information on the operation area and the non-operation area to the reconstruction unit. The reconstruction unit is electrically connected to the input/output unit, and the reconstruction unit receives the MRI image, and the information on the operation area and the non-operation area, reconstructs the image according to the MRI image and the information on the operation area and the non-operation area and transmits the reconstructed image to the input/output unit. And the input/output unit is electrically connected to the workstation module 3, and the input/output unit receives the reconstructed image and transmits the reconstructed image to the workstation module 3.

In some specific embodiments of the present disclosure, the image acquisition unit is electrically connected to the analysis unit, the image acquisition unit is applied to acquiring the MRI image of the object, the analysis unit stores an image of a normal human body, and determines the operation area and the non-operation area by comparing the MRI image of the object with the image of the normal human body, the reconstruction unit is electrically connected to the image acquisition unit, the analysis unit and the input/output unit separately, the reconstruction unit performs image fusion and reconstructs the image according to the operation area, the non-operation area and the MRI image to acquire the reconstructed image highlighted of the operation area and the non-operation area, and transmits the reconstructed image to the workstation module 3 by the input/output unit. The planning module 1 may receive, by the input/output unit, an instruction from the workstation module 3, to be switched on and switched off, or a control unit may be arranged on the planning module 1 to control the planning module 1 to be switched on and switched off.

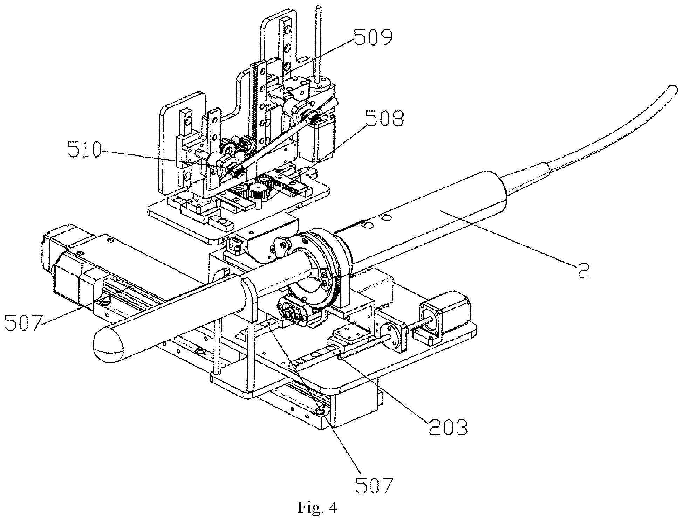

Fig. 4 is a schematic diagram of an assembling structure of an adjustment module and an ultrasonic module in a second embodiment of the present disclosure.

In some embodiments of the present disclosure, with reference to Figs. 1, 3 and 4, Fig. 4 differs from Fig. 3 in that the adjustment module 5 comprises a planar moving mechanism 507, a first angle adjustment mechanism 508, a second angle adjustment mechanism 509 and a second puncture needle guide 510, the planar moving mechanism 507, the first angle adjustment mechanism 508 and the second angle adjustment mechanism 509 being electrically connected to the workstation module 3 separately. The first angle adjustment mechanism 508 is arranged on the planar moving mechanism 507, the second angle adjustment mechanism 509 is arranged on the first angle adjustment mechanism 508, and the second moving mechanism 502 is perpendicular to the first moving mechanism 501, the planar moving mechanism 507 is applied to driving the first angle adjustment mechanism 508 to move, and the first angle adjustment mechanism 508 is applied to driving the second angle adjustment mechanism 509 to rotate, and the second angle adjustment mechanism 509 is applied to driving the second puncture needle guide 510 to rotate.

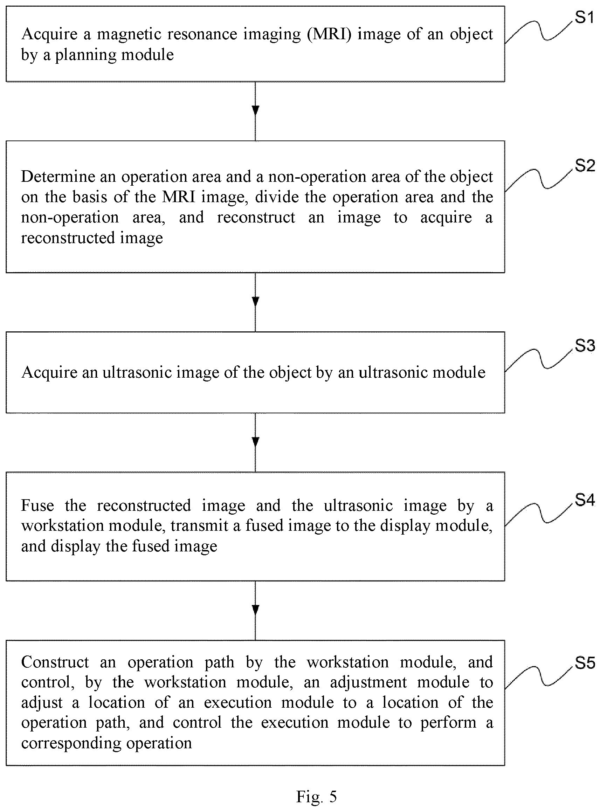

Fig. 5 is a flowchart of a control method for a puncture positioning system in a third embodiment of the present disclosure.

In some embodiments of the present disclosure, with reference to Figs. 1, 2, 3 and 5, the control method for the puncture positioning system comprises: S 1: a magnetic resonance imaging (MRI) image of an object is acquired by a planning module 1; S2: an operation area and a non-operation area of the object are determined on the basis of the MRI image, the operation area and the non-operation area are divided, and an image is reconstructed; S3: an ultrasonic image of the object is acquired by an ultrasonic module 2; S4: the reconstructed image and the ultrasonic image are fused by a workstation module 3, and a fused image is transmitted to a display module 4 and is displayed; and S5: an operation path is constructed by the workstation module 3, and the workstation module 3 controls an adjustment module 5 to adjust a location of an execution module 6 to a location of the operation path, and the execution module 6 is controlled to perform a corresponding operation.

In some specific embodiments of the present disclosure, before an operation, the planning module 1 acquires the MRI image of a portion to be operated of the object, and divides and reconstructs the image of a lesion on the basis of the MRI image. During operation, the ultrasonic module 2 is electrically connected to the workstation module 3, then the ultrasonic module 2 is switched on, the workstation module 3 controls a rotary mechanism 202 and/or a linear movement mechanism 203 to acquire an ultrasonic image of the portion to be operated of the object, and then to transmit the ultrasonic image and the reconstructed image to the workstation module 3, the workstation module 3 determines a non-operation area of the portion to be operated on the basis of the ultrasonic image, and constructs an operation path according to the non-operation area and the operation area. In this case, the ultrasonic image and the reconstructed image are displayed by the display module 4, and the workstation module 3 controls the adjustment module 5 to be switched on and switched off to adjust the location of the execution module 6 to the operation path, and then the workstation module 3 controls the execution module 6 to be switched on to puncture.

In some embodiments of the present disclosure, with reference to Figs. 1, 2 and 5, S5 specifically comprises: S51: several operation paths are constructed by the workstation module 3; S52: one of the operation paths is selected in S51 by the workstation module 3; S53: the adjustment module 5 is controlled to adjust the location of the execution module 6 to a location of the selected operation path; S54: the corresponding operation is performed by the execution module 6; and S55: steps S52-S54 are repeated until all operations are completed.

In some specific embodiments of the present disclosure, since puncture is often applied to acquiring pathological samples from different directions in the operation area for many times, accuracy of case analysis is improved. By means of the workstation module 3, the plurality of operation paths are constructed for selection, then one operation path is selected, the location of the execution module 6 is adjusted to the location of the selected operation path, the corresponding operation is performed by the execution module 6, and after that, another operation path is selected for the corresponding operation again, such that different pathological samples may be acquired.

In some embodiments of the present disclosure, with reference to Figs. 1, 2 and 5, S53 specifically comprises: S530: a location module is provided, where the location module is arranged on the execution module 6, and the location module acquires a location of the execution module 6 in real time and transmits the location to the workstation module 3 and the display module 4; and S531: the workstation module 3 computes adjustment and an adjustment distance required by the execution module 6 according to the real-time location and the selected operation path, the adjustment module 5 is controlled to be switched on according to the adjustment, the adjustment module 5 is controlled to be switched off when the adjustment distance is zero, and the location is received and displayed in real time by the display module 4.

In some specific embodiments of the present disclosure, the location of the execution module 6, specifically the execution unit, is acquired by the location module, and the location and an angle of the execution module 6 in space are determined, transmitted to the display module 4 for observation, and transmitted to the workstation module 3, such that the workstation module 3 may automatically control the adjustment module 5 to be switched on and switched off according to the location and the location of the selected operation path.

In some embodiments of the present disclosure, with reference to Figs. 1, 2 and 5, S54 specifically comprises: S540: with the execution module 6 comprising an execution unit and a drive unit, the execution unit is arranged at a movable end of the drive unit, the execution unit is applied to performing the corresponding operation, the drive unit is applied to driving the execution unit to move, and the workstation module 3 is applied to controlling the drive unit to be switched on and switched off; S541: the workstation module 3 controls the drive unit to be switched on; S542: an initial location of the execution unit is acquired by the location module, and the workstation module 3 computes a required traveling depth of the execution unit according to the initial location and the operation area; S543: a real-time traveling distance of the execution unit is acquired by the location module, and the drive unit is controlled to be switched off when the traveling distance is equal to the depth; S544: the corresponding operation is performed by the execution unit; and S545: the workstation module 3 controls the drive unit to drive the execution unit to exit.

In some specific embodiments of the present disclosure, the workstation module 3 controls the drive unit to be switched on, drives the execution unit to travel along the operation path, acquires the initial location of the execution unit by means of the location module, computes the required traveling depth of the execution unit by means of the operation area, and acquires the traveling distance of the execution unit in real time, the workstation module 3 controls the drive unit to be switched off when the depth is equal to the traveling distance, in this case, the execution unit just moves to the operation area, then the pathological samples of the operation area may be acquired by performing the corresponding operation by the execution unit, then, the workstation module 3 controls the execution unit to exit and be switched off, such that the execution module 6 may be automatically controlled to be switched on and switched off conveniently.

In some embodiments of the present disclosure, with reference to Figs. 1, 2 and 5, S3 specifically comprises: S31: the ultrasonic module 2 is switched on; S32: the ultrasonic module 2 is moved to an area to be operated of the object; and S33: the ultrasonic module 2 is automatically operated to acquire the ultrasonic image.

In some specific embodiments of the present disclosure, the ultrasonic module 2 is arranged on the connection module 8, the workstation module 3 and the ultrasonic module 2 are connected by means of a cable, then, the ultrasonic module 2 is switched on, then the ultrasonic module 2 is moved to the area to be operated of the object by means of the connection module 8, a location of the ultrasonic probe 201 is adjusted to be centred, and then the ultrasonic module 2 is automatically controlled to operate to drive the ultrasonic probe 201 to rotate or move back and forth to acquire the ultrasonic image, such that an acquired image is more accurate and manual operation may be omitted.

In some embodiments of the present disclosure, with reference to Figs. 1, 2 and 5, after S3 and before S4, the control method further comprises: the non-operation area is divided and reconstructed by the ultrasonic module 2 according to the ultrasonic image acquired in S3.

In some specific embodiments of the present disclosure, the ultrasonic module 2 acquires the ultrasonic image of the object, divides and reconstructs the ultrasonic image, acquires an image highlighted of the non-operation area of the object in a current state, and transmits the image to the workstation module 3 and the display module 4, such that the operator may conveniently perform observation, establish a safe operation path or correct an original operation path.

Although the embodiments of the present disclosure have been described in detail above, it is apparent to those skilled in the art that various modifications and changes can be made to these embodiments. However, it should be understood that such modifications and changes shall fall within the scope and spirit of the present disclosure described in the claims. Moreover, the present disclosure described herein can have other embodiments and can be performed or implemented in various ways.

## Claims

1. A puncture positioning system, comprising a planning module, an ultrasonic module, a workstation module, a display module, an adjustment module and an execution module, wherein
the planning module is electrically connected to the workstation module, the planning module is applied to acquiring a magnetic resonance imaging (MRI) image of an object and determining an operation area and a non-operation area according to the MRI image, the planning module reconstructs an image according to the operation area, the non-operation area and the MRI image, and the planning module transmits the reconstructed image to the workstation module;
the ultrasonic module is electrically connected to the workstation module, and the ultrasonic module is applied to acquiring an ultrasonic image of the object and transmitting the ultrasonic image to the workstation module;
the workstation module is electrically connected to the display module, the workstation module receives the reconstructed image and the ultrasonic image and constructs an operation path, the workstation module transmits the reconstructed image and the ultrasonic image to the display module, and the display module receives and displays the reconstructed image and the ultrasonic image; and
both the adjustment module and the execution module are electrically connected to the workstation module, the workstation module is applied to controlling the adjustment module and the execution module to be switched on and switched off, the execution module is arranged on the adjustment module, the adjustment module is applied to adjusting a location and an angle of the execution module, and the execution module is applied to performing a corresponding operation.

2. The puncture positioning system according to claim 1, further comprising a location module, wherein
the location module is arranged on the execution module, the location module is electrically connected to the workstation module, and the location module is applied to acquiring a location of the execution module and transmitting the location to the workstation module.

3. The puncture positioning system according to claim 2, further comprising a fusion module and a computation module, wherein
the fusion module is electrically connected to the workstation module, the display module and the computation module separately, the fusion module acquires the location of the execution module and the operation path and fuses the location and the operation path into a real-time location, and the fusion module transmits the real-time location to the computation module and the display module; and
the computation module is electrically connected to the workstation module, and the computation module receives the real-time location, computes a difference between the location of the execution module and the operation path according to the real-time location, and transmits the difference to the workstation module.

4. The puncture positioning system according to claim 1, further comprising a moving module and a connection module;
the workstation module and the display module are arranged on the moving module, and the moving module is applied to adjusting a location; and
the connection module is arranged on the moving module, the ultrasonic module and the adjustment module are arranged on the connection module, and the connection module is applied to adjusting a location of the ultrasonic module and a location of the adjustment module.

5. The puncture positioning system according to claim 4, wherein the moving module is provided with a hollowed inner cavity, the hollowed inner cavity is applied to storing the connection module, the ultrasonic module, the adjustment module and the execution module.

6. The puncture positioning system according to claim 1, wherein the planning module comprises an image acquisition unit, an analysis unit, a reconstruction unit and an input/output unit;
the image acquisition unit is electrically connected to the analysis unit and the reconstruction unit separately, and the image acquisition unit is applied to acquiring the MRI image and transmitting the MRI image to the analysis unit and the reconstruction unit;
the analysis unit is electrically connected to the reconstruction unit, the analysis unit receives the MRI image and determines the operation area and the non-operation area, and the analysis unit transmits information on the operation area and the non-operation area to the reconstruction unit;
the reconstruction unit is electrically connected to the input/output unit, and the reconstruction unit receives the MRI image, and the information on the operation area and the non-operation area, reconstructs the image according to the MRI image and the information on the operation area and the non-operation area and transmits the reconstructed image to the input/output unit; and
the input/output unit is electrically connected to the workstation module, and the input/output unit receives the reconstructed image and transmits the reconstructed image to the workstation module.

7. The puncture positioning system according to any one of claims 1-6, wherein the ultrasonic module comprises an ultrasonic probe, a rotary mechanism, a linear movement mechanism and an ultrasonic sheath;
the ultrasonic probe is arranged on the rotary mechanism, the rotary mechanism is applied to driving the ultrasonic probe to rotate, and the ultrasonic probe is applied to acquiring the ultrasonic image;
the ultrasonic sheath is arranged on one side of the ultrasonic probe, the rotary mechanism is arranged on the linear movement mechanism, and the linear movement mechanism is applied to driving the ultrasonic probe to reciprocate; and
the ultrasonic probe, the rotary mechanism and the linear movement mechanism are electrically connected to the workstation module, the ultrasonic probe transmits the ultrasonic image to the workstation module, and the workstation module controls the ultrasonic probe, the rotary mechanism and the linear movement mechanism to be switched on and switched off separately.

8. The puncture positioning system according to claim 7, wherein the adjustment module comprises a first moving mechanism, a second moving mechanism, a first fixing clip, a second fixing clip, a third moving mechanism and a first puncture needle guide;
the first moving mechanism is arranged on the rotary mechanism, the second moving mechanism is arranged on the first moving mechanism, the first moving mechanism and the second moving mechanism are arranged with an angle defined therebetween, and the first moving mechanism is applied to driving the second moving mechanism to move;
the first fixing clip and the third moving mechanism are arranged on the second moving mechanism, and the second moving mechanism is applied to driving the first fixing clip and the third moving mechanism to move;
the second fixing clip is arranged on the third moving mechanism, and the third moving mechanism is applied to driving the second fixing clip to rotate around the first fixing clip;
the first puncture needle guide is arranged on the first fixing clip and the second fixing clip, and the execution module is arranged on the first puncture needle guide or the third moving mechanism; and
the first moving mechanism, the second moving mechanism and the third moving mechanism are all electrically connected to the workstation module, and the workstation module controls the first moving mechanism, the second moving mechanism and the third moving mechanism to be switched on and switched off separately.

9. The puncture positioning system according to any one of claims 1-6, wherein the adjustment module comprises a planar moving mechanism, a first angle adjustment mechanism, a second angle adjustment mechanism and a second puncture needle guide, wherein
the first angle adjustment mechanism is arranged on the planar moving mechanism, the second angle adjustment mechanism is arranged on the first angle adjustment mechanism, the first angle adjustment mechanism and the second angle adjustment mechanism are arranged with an angle defined therebetween, the second puncture needle guide is arranged on the second angle adjustment mechanism, the planar moving mechanism is applied to driving the first angle adjustment mechanism to move, the first angle adjustment mechanism is applied to driving the second angle adjustment mechanism to rotate, and the second angle adjustment mechanism is applied to driving the second puncture needle guide to rotate; and
the planar moving mechanism, the first angle adjustment mechanism and the second angle adjustment mechanism are each electrically connected to the workstation module, and the workstation module controls the planar moving mechanism, the first angle adjustment mechanism and the second angle adjustment mechanism to be switched on and switched off separately.

10. A control method for the puncture positioning system according to claim 1, comprising:
S1: acquiring a magnetic resonance imaging (MRI) image of an object by a planning module;
S2: determining an operation area and a non-operation area of the object on the basis of the MRI image, dividing the operation area and the non-operation area, and reconstructing an image to acquire a reconstructed image;
S3: acquiring an ultrasonic image of the object by an ultrasonic module;
S4: fusing the reconstructed image and the ultrasonic image by a workstation module, transmitting a fused image to a display module, and displaying the fused image by the display module; and
S5: constructing an operation path by the workstation module, and controlling, by the workstation module, an adjustment module to adjust a location of an execution module to a location of the operation path, and controlling the execution module to perform a corresponding operation.

11. The control method for the puncture positioning system according to claim 10, wherein S5 specifically comprises:
S51: constructing several operation paths by the workstation module;
S52: selecting one of the operation paths in S51 by the workstation module;
S53: controlling the adjustment module to adjust the location of the execution module to a location of the selected operation path;
S54: performing the corresponding operation by the execution module; and
S55: repeating steps S52-S54 until all operations are completed.

12. The control method for the puncture positioning system according to claim 11, wherein S53 specifically comprises:
S530: providing a location module, wherein the location module is arranged on the execution module, and the location module acquires a location of the execution module in real time and transmits the location to the workstation module and the display module; and
S531: computing, by the workstation module, adjustment and an adjustment distance required by the execution module according to the real-time location and the selected operation path, controlling the adjustment module to be switched on according to the adjustment, controlling the adjustment module to be switched off when the adjustment distance is zero, and receiving and displaying in real time the location by the display module.

13. The control method for the puncture positioning system according to claim 12, wherein S54 specifically comprises:
S540: with the execution module comprising an execution unit and a drive unit, arranging the execution unit at a movable end of the drive unit, wherein the execution unit is applied to performing the corresponding operation, the drive unit is applied to driving the execution unit to move, and the workstation module is applied to controlling the drive unit to be switched on and switched off;
S541: controlling, by the workstation module, the drive unit to be switched on;
S542: acquiring an initial location of the execution unit by the location module, and computing, by the workstation module, a required traveling depth of the execution unit according to the initial location and the operation area;
S543: acquiring a real-time traveling distance of the execution unit by the location module, and controlling the drive unit to be switched off when the traveling distance is equal to the depth;
S544: performing the corresponding operation by the execution unit; and
S545: controlling, by the workstation module, the drive unit to drive the execution unit to exit.

14. The control method for the puncture positioning system according to claim 10, wherein S3 specifically comprises:
S31: switching on the ultrasonic module;
S32: moving the ultrasonic module to an area to be operated of the object; and
S33: automatically operating the ultrasonic module to acquire the ultrasonic image.

15. The control method for the puncture positioning system according to claim 10, wherein after S3 and before S4, the control method further comprises: dividing and reconstructing, by the ultrasonic module, the non-operation area according to the ultrasonic image acquired in S3.
